# EUROPEAN PATENT SPECIFICATION

(11) **EP 1 711 597 B1**
(45) Date of publication and mention of the grant of the patent: **07.03.2012**
(21) Application number: 05708747.0
(22) Date of filing: 14.01.2005
(51) Int. Cl.: C12N 5/071, A61K 35/407, C12Q 1/02, A61P 1/16

(54) **HUMAN HEPATIC PROGENITOR CELLS AND METHODS OF USE THEREOF**
MENSCHLICHER HEPATISCHE VORLÄUFERZELLEN UND VERFAHREN ZUR VERWENDUNG DAVON
CELLULES PROGENITRICES HEPATIQUES HUMAINES ET PROCEDES D'UTILISATION CORRESPONDANTS

(30) Priority: 14.01.2004 US 536405 P; 27.10.2004 US 623003 P
(43) Date of publication of application: 18.10.2006
(73) Proprietor: Novahep AB, 103 95 Stockholm (SE)
(72) Inventor: HOLGERSSON, Suchitra, S-141 33 Huddinge (SE)
(74) Representative: Peter, Beate
(86) International application number: PCT/IB2005/000660
(87) International publication number: WO 2005/068612

(56) References cited:
- STRAIN A J ET AL: "Human liver-derived stem cells." SEMINARS IN LIVER DISEASE, vol. 23, no. 4, November 2003 (2003-11), pages 373-384, XP008057005 ISSN: 0272-8087
- BAUMANN U ET AL: "Expression of the stem cell factor receptor c-kit in normal and diseased pediatric liver: Identification of a human hepatic progenitor cell?" HEPATOLOGY, vol. 30, no. 1, July 1999 (1999-07), pages 112-117, XP002358742 ISSN: 0270-9139
- LÁZARO C A ET AL: "Establishment, characterization, and long-term maintenance of cultures of human fetal hepatocytes." HEPATOLOGY, vol. 38, no. 5, November 2003 (2003-11), pages 1095-1106, XP002322701 ISSN: 0270-9139
- GERLACH J C ET AL: "Use of primary human liver cells originating from discarded grafts in a bioreactor for liver support therapy and the prospects of culturing adult liver stem cells in bioreactors: A morphologic study." TRANSPLANTATION, vol. 76, no. 5, 15 September 2003 (2003-09-15), pages 781-786, XP002358743 ISSN: 0041-1337
- CROSBY H A ET AL: "Human hepatic stem-like cells isolated using c-kit or CD34 can differentiate into biliary epithelium." GASTROENTEROLOGY, vol. 120, no. 2, February 2001 (2001-02), pages 534-544, XP002358744 ISSN: 0016-5085
- GUETTIER C: "Quelle(s) cellule(s) souche(s) pour la régénération du foie adulte ?" ANNALES DE PATHOLOGIE, vol. 25, no. 1, February 2005 (2005-02), pages 33-44, XP008057007 ISSN: 0242-6498
- NAVA S ET AL: "Characterization of cells in the developing human liver." DIFFERENTIATION, vol. 73, no. 5, June 2005 (2005-06), pages 249-260, XP002358746 ISSN: 0301-4681

## Description

### FIELD OF THE INVENTION

The invention relates to progenitor cells.

### BACKGROUND OF THE INVENTION

Acute liver failure remains an important problem with high mortality. Despite the high incidence of diseases that result in liver dysfunction and failure, major advances in medical therapies are currently limited to the prevention and treatment of certain forms of viral hepatitis. The acute and chronic liver diseases are still treated with supportive rather than curative approaches. Orthotopic liver transplantation has so far been the only available therapy for patients with end-stage liver failure. Unfortunately, the availability of donor organs is limited and many patients die each year waiting for liver transplants. Recently, transplantation of healthy hepatocytes into diseased liver has been used as an alternative therapy. However, the shortage of organ donors has limited the clinical application of hepatocyte cell transplantation.

Cellular therapy with stem cells and their progeny is a promising new approach to the largely unmet medical need for patients with liver diseases. A number of studies have examined the potential of stem/progenitor cell (obtained from extra-hepatic and intra-hepatic tissues) transplantation in experimentally induced acute liver failure. Although stem/progenitor cells from adult organs may generate functional liver cells, such cells are rare. Clinical therapeutic protocols involving hepatic progenitor cell transplantation to ameliorate inherited and acquired disease would greatly benefit by the capability to produce large numbers of these cells that still retain their complete definitive functions.

Thus, a need exists for a source of liver progenitor cells that can differentiate into functional liver cells.

### SUMMARY OF THE INVENTION

The invention is based on the discovery of a liver progenitor cell. The progenitor cell is multipotent. The progenitor cell is capable of differentiating *in vivo* into a hepatocyte, a cholangiocyte or a liver endothelial cell (ie a sinusoidal cell). Accordingly, the invention provides an isolated cell population as defined in claim 1. The population is an adhesion culture. Alternatively, the cells in the population are in suspension. The cell is derived from liver tissue, such as fetal liver tissue. The tissue is from a human. The cell is immunoreactive for CD117 and CD34 and non-immunoreactive with Lin. The cells proliferate *in vitro.* The cells are capable of doubling 2, 3, 4, 5, 6, 7, 8, 9, 10, 11, 12, 15, 20, 25 or more times and maintain their ability to differentiate into hepatocytes, cholangiocytes or liver endothelial cells.

Also provided are methods of producing a human liver hepatocyte or cholangiocyte or a human liver sinusoidal cell by differentiating the liver progenitor cell cultures in a culture medium containing one or more differentiation factors as defined in claims 5 and 6. Differentiation factors include vascular endothelial growth factor (VEGF), hepatocyte growth factor (HGF) and epidermal growth factor (EGF).

The invention further features use of a multipotent liver progenitor cell which is CD 117⁺, CD34⁺, CD45⁻ and Lin⁻ in the manufacture of a composition for transplanting into a host, eg mammal such as human, a primate, mouse, rat, dog, cat, cow, horse, pig. Optionally, fetal liver stromal or fetal liver mesenchymal cells are co-transplanted in the host. Use of cells derived from human embryos is not encompassed. The host is administered hepatocyte growth factor prior to, after or concomitantly with the progenitor cells. The host is suffering from a hepatic disorder or hepatic tissue damage. For example, the subject is suffering from hepatitis, cirrhosis, liver cancer, fatty liver disease, Reye syndrome, glycogen storage disease, liver cysts or Wilson's disease. Transplantation confers a clinical benefit, eg alleviating one or more symptoms of the particular liver disorder. Liver disorders are diagnosed by a physician using methods known in the art.

Compounds which effect proliferation, differentiation or survival of liver cells are identified by contacting the liver progenitor cell culture with a test compound and determining of the compound has an effect on proliferation, differentiation or survival of the cells. Similarly, the metabolite of a test compound is determined. Metabolites are identified by screening the culture medium after contacting the culture with the test compound.
Metabolites are identified by methods know in the art such a HPLC, Mass spectroscopy or gel electrophoresis. Anti-viral activity of a test compound is determined by introducing a virus to a progenitor cell culture in the presence or absence of a test compound and determining the survival rate of the cells. An increase in survival rate in the presence of the test compound compared to the absence of the test compound indicates that the test compound has anti-viral activity. Infectivity of a virus is determined by contacting a progenitor cell culture with a virus and determining the effect on proliferation or survival of the cells. A decrease of survival or proliferation as compared to a cell culture that has not been contacted with the virus indicate the virus can infect liver cells. In contrast, a similarity of survival or proliferation as compared to a cell culture that has not been contacted with the virus indicate the virus does not infect liver cells.

Optionally, the culture is differentiated prior to contacting the culture with a test compound. Proliferation and or survival is determined by methods know in the art such as BrdU assay. Differentiation is determined morphologically or histologically by determining hepatic cell surface markers. The virus is a liver trophic virus such as hepatitis virus A, hepatitis virus B, or hepatitis virus C.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and not intended to be limiting.

Other features and advantages of the invention will be apparent from the following detailed description and claims.

### BRIEF DESCRIPTION OF THE DRAWINGS

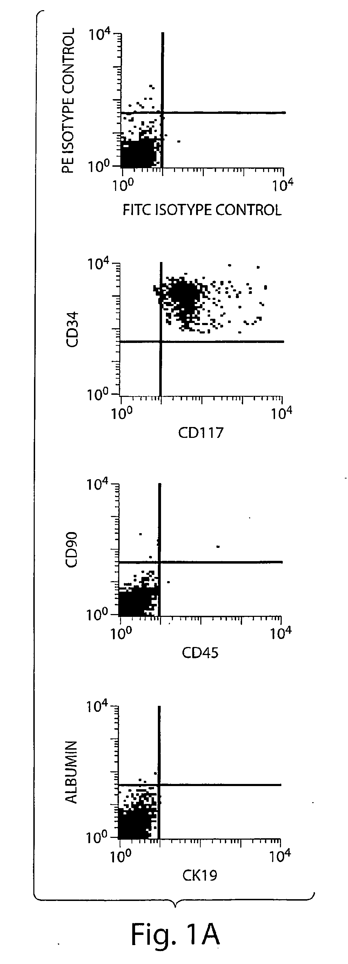

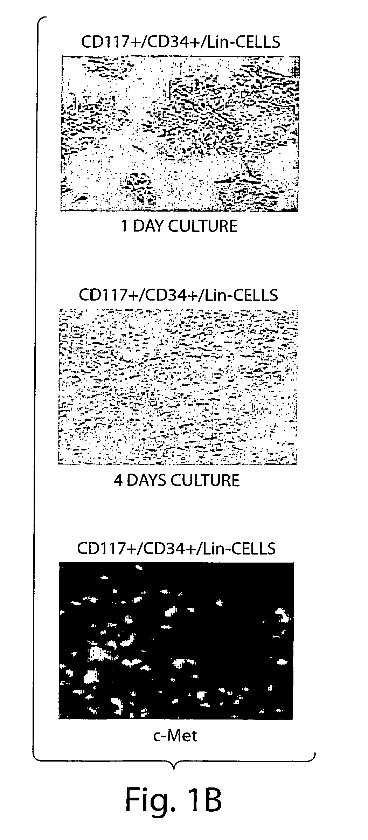

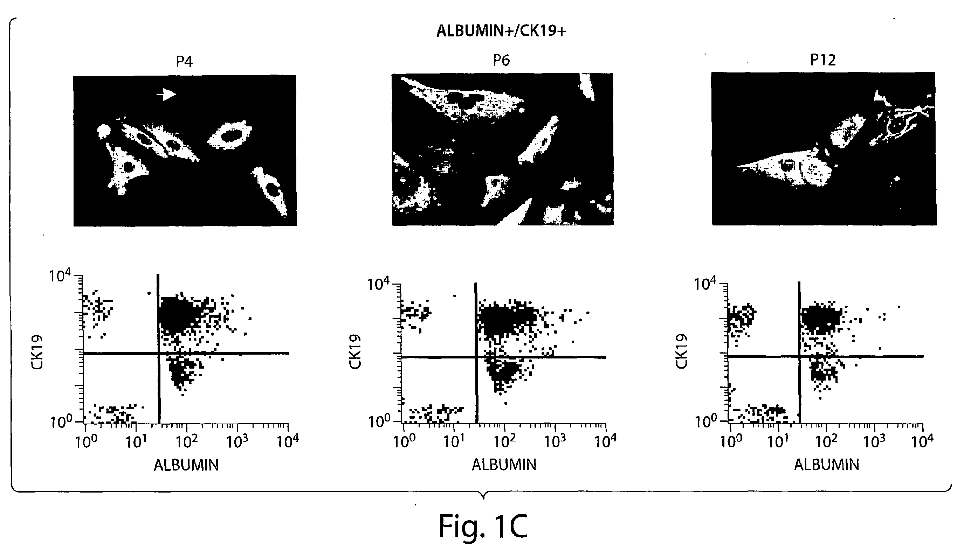

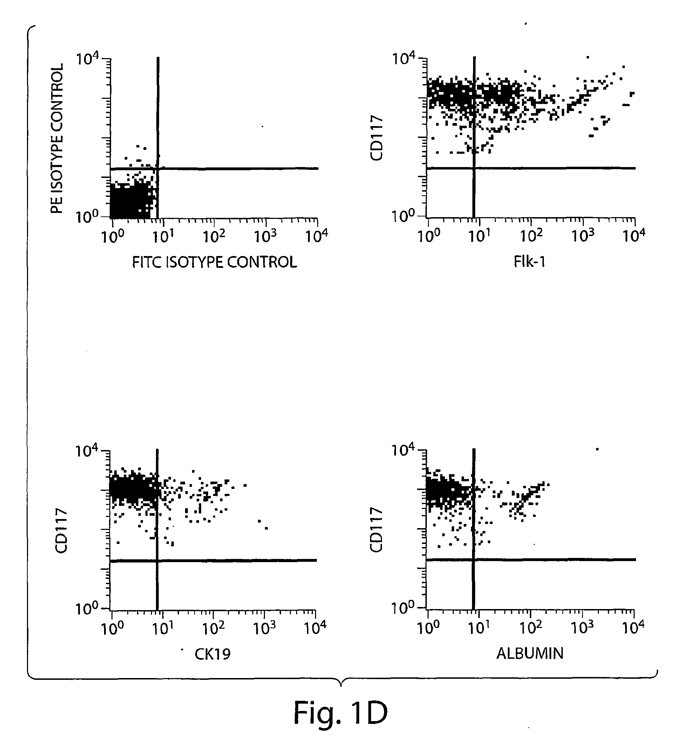

Fig. 1 is a series of FACS analysis and photographs showing the characterization of human hepatic progenitor cells a, Magnetically sorted FL cells immediately after isolation were double-stained for CD117 and CD34, but negative for CD90, CD45, albumin and cytokeratin 19 (CK19). b, CD117+/CD34+/Lin- cells when cultured grew in colonies and the first marker to be expressed was the hepatocyte growth factor receptor (c- Met). Magnification 40x. c, Freshly isolated and expanded cells in various passages gave rise to ALB-CK19-(white arrow), ALB+(green)CK19+(red), ALB+CK19-(green, hepatocytes) and ALB-CK19+(red, cholangiocytes) Magnification 60x. d, Magnetically sorted CD117+/CD34+/Lin- adherent cells when grown in culture medium containing vascular endothelial growth factor differentiated into Flk-1+ endothelial cells (~50%), CK19+ cholangiocytes (~13%) and albumin+ hepatocytes (~17%). Approximately 20% cells did not express any of these markers.
Fig. 2A is a bar chart showing proliferation of hepatic progenitors and their progeny. Use of 20% conditioned medium (CM) significantly increased the proliferation of hepatic progenitor cells and' could be passaged several times as compared to without CM (p<0.001, students t test).
Figure 2B is a bar chart showing the results of flow cytometric analysis demonstrating that, a high proliferation (BrdU+ cells) was observed in albumin+CK19+, albumin+CK19- and albumin-CK19+ cells, while, double negative (albumin- CK19-) cells were more quiescent.
Figure 2C is a series of photographs showing liver progenitor cells in various passages, fluorescence stained for albumin (green) and CK19 (red) and enzymatically for detection of BrdU incorporation, showing proliferative capacity in the various subpopulations. Magnification 40x.

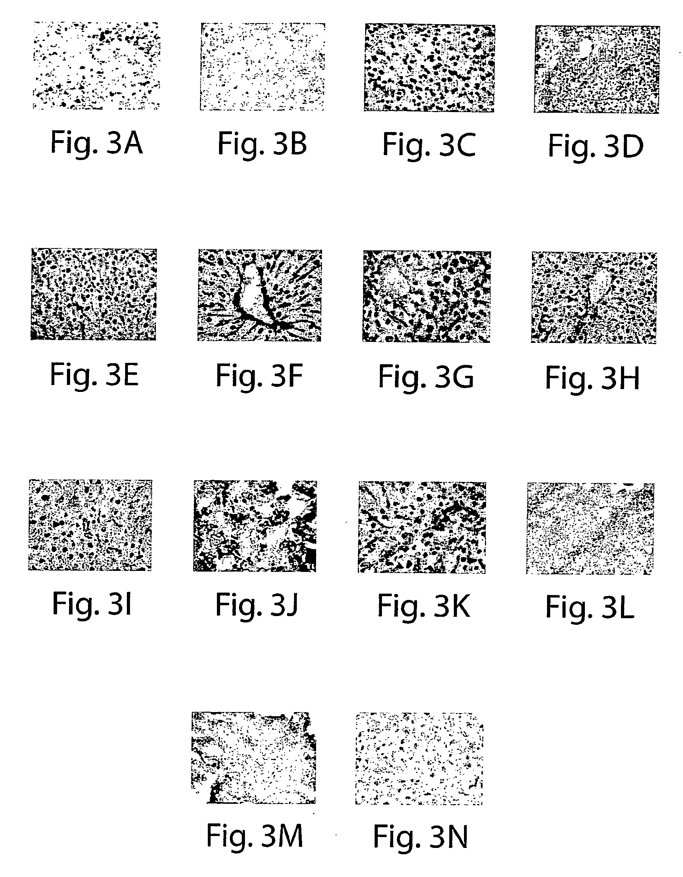

Figure 3A- 3N are a series of photographs Localization of human hepatic progenitor cells in the mouse liver. **a-b**, In situ hybridization with human centromere probe, showing nuclear signals in human liver (DAB) but not in the mouse liver. **c-d**, using immunohistochemistry, similar results were obtained with an anti-human nuclei antibody (DAB-Ni). **e-j,** Freshly isolated cells **(e-g),** sixth and twelfth passage cells when transplanted into D-galactosamine-treated (GalN) mice showed differentiation into hepatocytes cholangioytes and endothelial cells (DAB-Ni- arrow heads). **k,** Transplanted cells were observed in the livers, **l-m,** but not in the spleens, lungs of GalNtreated mice. **n,** Livers of sham-transplanted mice were used as controls. Magnification 60x.

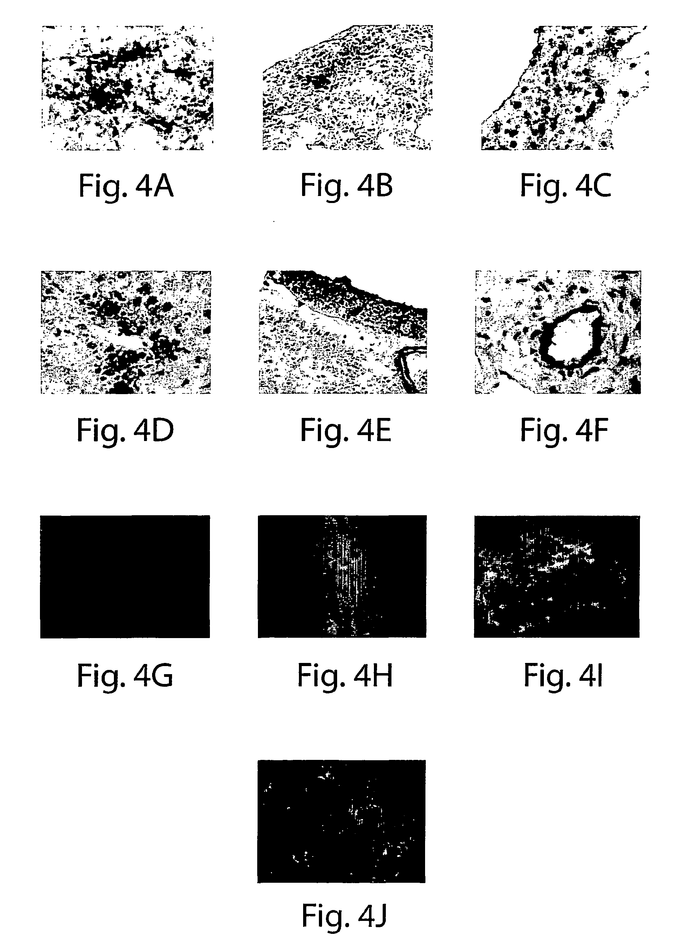

Fig. 4A-J is a series of photographs showing the in vivo fate of human hepatic progenitor cells. **a-d,** Human hepatic progenitor cells one month after transplantation into GalN-treated mice contained glucose-6-phosphatase (brown), glycogen (pink), dipeptidyl peptidase IV (red-brown), gamma glutamyl transpeptidase (brown). Double-staining with the anti-human nuclei antibody (DAB-Ni, arrow heads) visualised human nuclei (black). **e,** Regeneration of a whole mouse tissue segment by human hepatic progenitors (black nucleic, arrow heads). **f**, Expression of cytokeratin 19 (red-brown) and g-**j**, human albumin (green) was not observed in the sham-transplanted mice but was observed (arrow heads) in the livers of GaIN-treated mice that were transplanted with freshly isolated, sixth passage and twelfth passage human hepatic progenitor cells. **a-j**, (Magnification 60x). **f**, (Magnification 200x).
Figure 4K is a photograph showing transcription of human liver-specific genes in the mouse liver. Human cytokeratin 19, α-fetoprotein and albumin were detected in the livers of GalN-treated mice that received human hepatic progenitor cells but not in the sham-transplanted mice. However, α1 antitrypsin was slightly amplified also in the control. Glucose-6-phosphate dehydrogenase was used as the housekeeping gene.

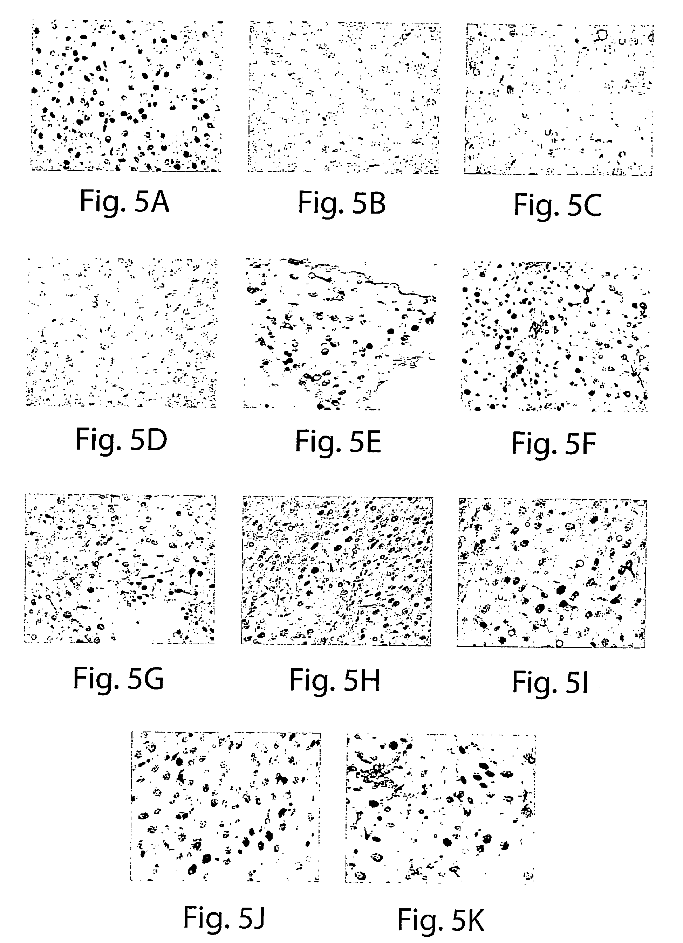

Figure 5 is a series of photographs demonstrating the effects of co-transplantion of fetal liver stromal cells and fetal liver progenitor cells. **A,** Normal human liver section stained with anti-human nuclei antibody shows positive staining (black/brown). **B,** Normal mouse liver section showed no positive staining with the same antibody, demonstrating the specificity of the antibody. **C,** Mice treated with retrorsine (30mg/kg), followed by partial hepatectomy and subcutaneously injected with the hepatocyte growth factor (HGF) followed by fetal liver progenitor cell transplantation did not result in high engraftment of cells. **D,** Mice treated with retrorsine (30mg/kg), followed by partial hepatectomy and injected withe stromal cells isolated from fetal livers did not result in high engraftment of cells. **E-H,** Mice treated with retrorsine (30mg/kg), followed by partial hepatectomy and transplanted with a mixture of fetal liver stromal and progenitor/stem cells resulted in high engraftment of stem cells. **I-K,** Mice treated with retrorsine (30mg/kg), followed by partial hepatectomy and subcutaneously injected with the hepatocyte growth factor (HGF) followed by fetal liver stromal and progenitor cell transplantation resulted in high engraftment of cells.
Figure 6 is a chart showing the detection levels of human factor VIII in normal nude C57 black mice and C57 black mice treated in various ways.

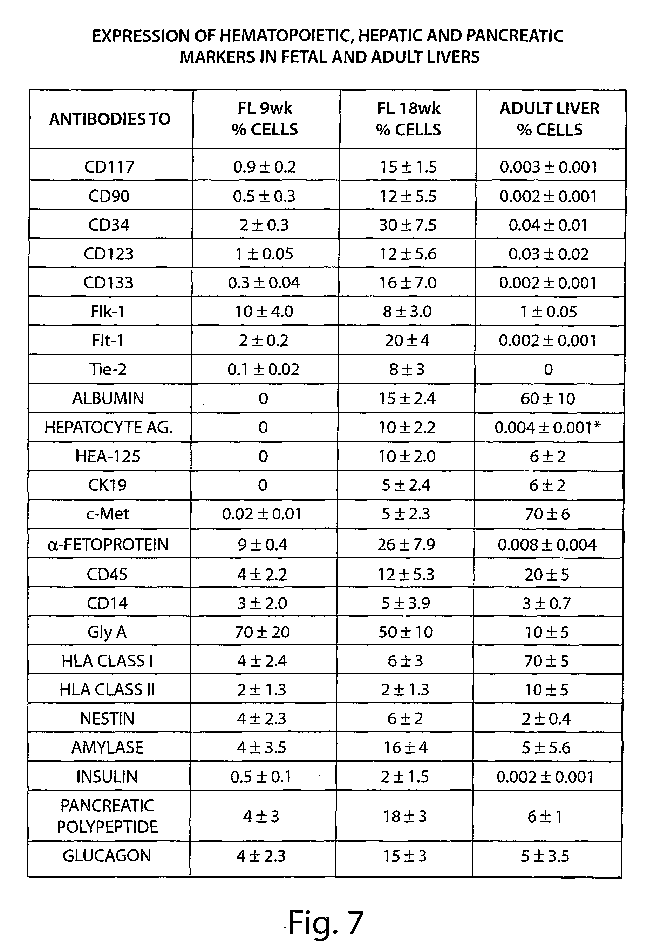

Figure 7 is a chart showing expression of various hematopoietic, hepatic and pancreatic cell surface markers on fetal and adult livers.

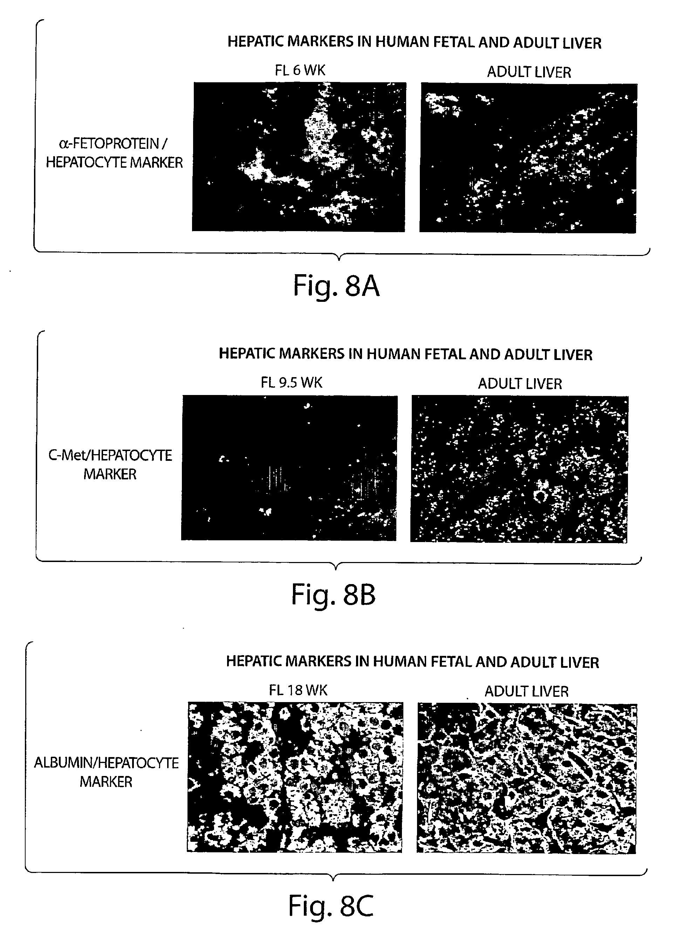

Figure 8 is a series of photographs showing hepatic markers in human fetal and adult liver.

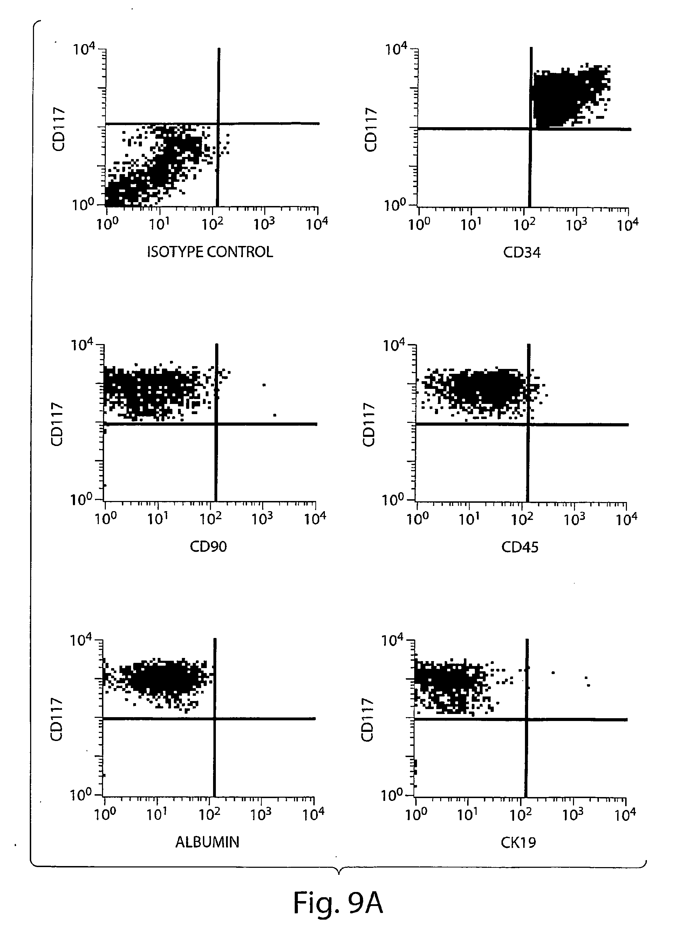

Figure 9A is a series of FACS analysis showing the characterization of human hepatic progenitor cells.

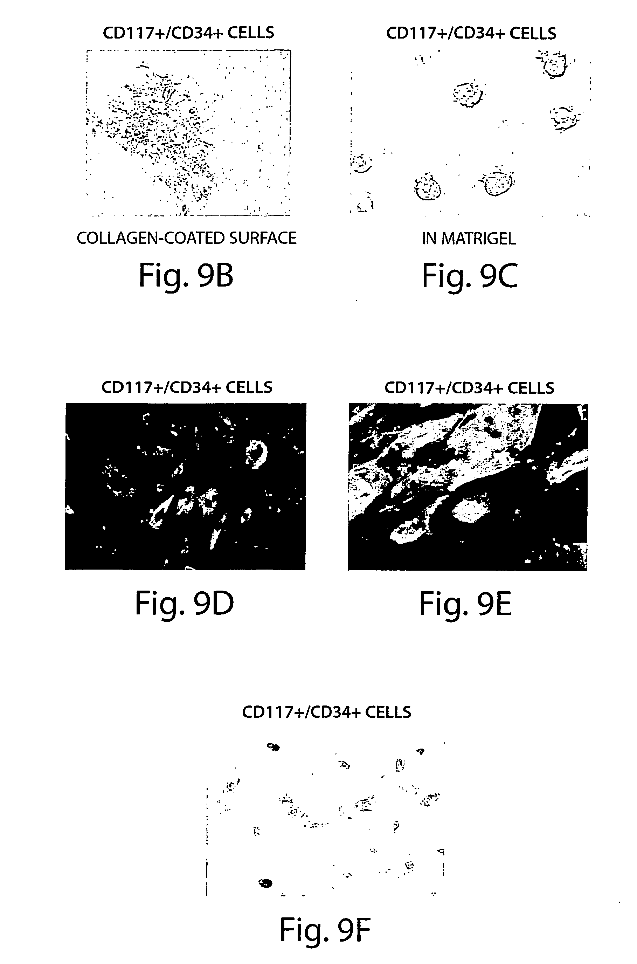

Figure 9B-F is a series of photographs showing morphology of human hepatic progenitor cells on different matrixes.

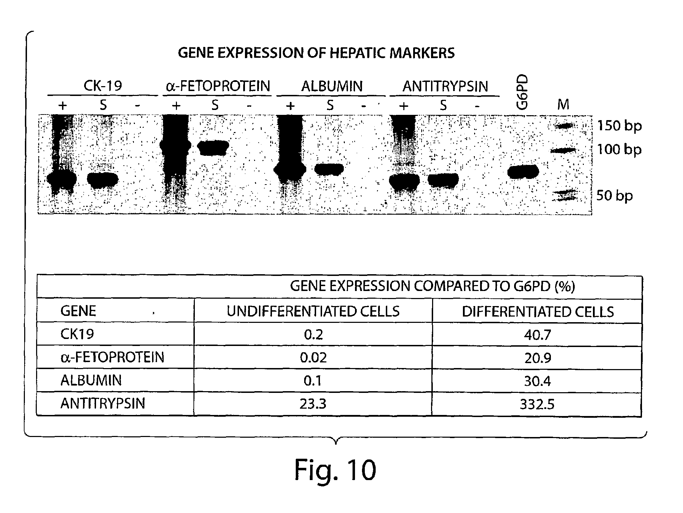

Figure 10A is a photograph of a NorthernBlot showing gene expression of hepatic markers in human hepatic progenitor cells.
Figure 10B is a chart demonstrating gene expression of hepatic markers in human hepatic progenitor cells.

### DETAILED DESCRIPTION

The present invention is based upon the unexpected discovery of a defined population of non-hematopoietic progenitor cells within human fetal liver that expand *in vitro* for several passages and maintain a progenitor phenotype. These cells, when transplanted into animals with acute liver injury exhibited functional differentiation into hepatocytes, cholangiocytes and sinusoidal cells.

Liver diseases include a wide spectrum of both acute and chronic conditions associated with significant morbidity and mortality worldwide. Hepatocyte transplantation has tremendous therapeutic potential in the treatment of liver diseases, but its clinical use is hampered by the lack of donor tissue. Generation of hepatocytes *in vitro* from adult or fetal liver cell progenitors, or identification of a progenitor population which *in vivo* can generate mature liver cells would solve this problem. The data described herein demonstrate the identification of a defined population of cells from human fetal livers that are successfully expanded *ex vivo* for several passages and when transplanted into animals with acute liver injury exhibited functional differentiation into hepatocytes, cholangiocytes and sinusoidal cells. Successful *in vitro* expansion and differentiation of liver progenitor cells are useful for hepatic cell transplantation, metabolic and toxicity testing of candidate therapeutic drugs, and a vehicle for gene therapy.

The present invention provides methods for inducing multipotent human hepatic progenitor cells from human fetal liver tissue to proliferate *in vitro,* to generate large numbers of multipotent human progenitor cell progeny capable of differentiating into hepatocytes, cholangiocytes and sinusoidal cells. Methods for differentiation of the human hepatic progenitor cells progeny are also provided.

### Human Liver Progenitor Cells

The invention provides a cell population consisting of human liver progenitor cells (referred to herein as LPC) as defined in claim 1. A LPC cell is an undifferentiated cell that can be induced to proliferate using the methods of the present invention. The LPC is capable of self-maintenance, such that with each cell division, at least one daughter cell will also be a LPC cell. LPC are capable of being expanded 100, 250, 500, 1000, 2000, 3000, 4000, 5000. or more fold.

Phenotyping of LPCs reveal that these cells do not express any committed hematopoietic markers, however the cells express stem cell markers. For example, a LPC is immunoreactive for both CD117 and CD34, and non immunoractive for Lineage surface antigen (Lin). The LPC is a multipotent progenitor cell. By mutipotent progenitor cell is meant that the cell is capable of differenctiating into more that one cell type. For example, the cell is capable of differentiating into a hepatocyte, acholangiocyte or a sinusoidal cell.

CD117 is also known as c-kit, steel factor receptor or stem cell factor receptor. CD117 is a e 145 kD cell surface glycoprotein belonging to the class III receptor tyrosine kinase family. It is expressed on the majority of hematopoietic progenitor cells, including multipotent hematopoietic stem cells as well' as on committed myeloid, erythroid and' lymphoid precursor cells. CD117 is also expressed on a few mature hematopoietic cells, e.g. mast cells. CD34 is a 110kD single chain transmembrane glycoprotein expressed on human lymphoid and myeloid hematopoietic progenitor cells. Lineage surface antigen is a mixture if Thirteen to 14 different cell-surface proteins that are markers of mature blood cell lineages

LPCs are obtained from embryonic liver tissue. The liver tissue can be obtained from. any animal that has liver tissue such as , fish, reptiles, birds, amphibians, and mammals, e.g. preferably rodents , and such as mice and humans. The tissue is obtained from a fetus that is at least 4, 5, 6, 7, 8, 9, 10 or more weeks of age. LPcs represent approximately 0.5-0.7% of whole fetal livers.

LPCs can be maintained *in vitro* in long-term cultures. The LPCs are capable of being passed in culture 2, 3, 4, 5, 6, 7, 8, 9, 10, 11, 12 or more times.

Prior to transplantation freshly isolated LPCs (CD117+/CD34+/Lin- cells) did not express albumin and CK19. However, after transplantation, these cells differentiated into mature hepatocytes and cholangiocytes as determined by the expression of human albumin CK19, G-6-P, GGT, DPPIV and glycogen and had high proliferative capacity. The liver progenitor cells expressed the two hematopoietic associated markers c-kit and CD34, but not CD45, the marker that distinguishes hematopoietic cells from non-hematopoietic cells. Thus, the LPCs with extensive proliferative capacity described herein are not of hematopoietic origin. Furthermore, unlike the limited ability to expand adult hematopoietic stem cells *in vitro* the hepatic progenitors from fetal livers have high proliferative capacity. The observation that not all of the isolated CD117+/CD34+/lin- cells adhered to the culture plate and differentiated to hepatic cells during in vitro cultivation indicates, that only a subpopul'ation of these cells are the progenitors of hepatic cells. The CD117+/CD34+/lin-cells when grown in medium containing HGF and EGF gave rise to four types of cells ALB-CK19-, ALB+CK19+, ALB+CK19- hepatocytes and ALB-CK19+ cholangiocytes. Except for the double negative cells all the other subpoulations had a high fraction of proliferating cell's (BrdU+). The ALB-CK19- cell subset were highly quiescent *in vitro,* however, *in vivo* the ten-fold increase in the number of cells transplanted in P0 passage indicate that these cells may have a high proliferative capacity. Despite the high proliferative capacity of liver progenitor cells no tumors were observed *in vivo* four weeks after human cell transplantation. Interestingly, CD117+/CD34+/Lin- cells from early fetal livers when grown in medium containing VEGF differentiated not only into hepatocytes and cholangiocytes but also into sinusoidal endothelial cells.

### Culture conditions

LPCs are proliferated using the methods described herein. Cells are obtained from donor tissue by dissociation of individual cells from the connecting extracellular matrix of the tissue. Tissue from fetuses are removed using a sterile procedure, and the cells are dissociated using any method known in the art including treatment with enzymes such as trypsin, collagenase and the like, or by using physical' methods of dissociation such as with a blunt instrument or homogenizer. Dissociation of fetal cells can be carried out in tissue culture medium.

For example, dissociation of cells can be carried out in 0.1% trypsin and 0.05% DNase in DMEM. Dissociated cells are centrifuged at low speed, between 200 and 2000 rpm, usually between 400 and 800 rpm, and then resuspended in a culture medium. The hepatic cells can be cultured in suspension or on a fixed substrate. Dissociated cell suspensions are seeded in any receptacle capable of sustaining cells, particularly culture flasks, culture plates or roller bottles, and more particularly in small culture flasks such as 25 cm² culture flasks. Cells cultured in suspension are resuspended at approximately 5 x 10⁴ to 2 x 10⁵ cells/ml (*for example,* 1 x 10⁵ cells/ml). Cells plated on a fixed substrate are plated at approximately 2-3 x 10³ 10 cells/cm². Optionally, the culture plates are coated with a matrix protein such as collagen. The dissociated hepatic cells can be placed into any known culture medium capable of supporting cell growth, including HEM, DMEM, RPMI, F-12, and the like, containing supplements which are required for cellular metabolism such as glutamine and other amino acids, vitamins, minerals and proteins such as transferrin and the like. The culture medium may also contain antibiotics to prevent contamination with yeast, bacteria and fungi such as penicillin, streptomycin, gentamicin and the like. The culture medium may contain serum derived from bovine, equine, chicken and the like.

Conditions for culturing should be close to physiological conditions. The pH of the culture medium should be close to physiological pH. (*for example,* between pH 6-8, between about pH 7 to 7.8, or at pH 7.4). Physiological temperatures range between about 30°C to 40°C. LPCs are cultured at temperatures between about 32°C to about 38°C (*for example,* between about 35°C to about 37°C).

Optionally, the culture medium is supplemented with at least one proliferation-inducing ("mitogenic") growth factor. A "growth factor" is protein, peptide or other molecule having a growth, proliferation-inducing, differentiation-inducing, or trophic effect on LPCs. "Proliferation-inducing growth factors" are trophic factor that allows LPCs to proliferate, including any molecule that binds to a receptor on the surface of the cell to exert a trophic, or growth-inducing effect on the cell. Proliferation-inducing growth factors include EGF, amphiregulin, acidic fibroblast growth factor (aFGF or FGF-1), basic fibroblast growth factor (bFGF or FGF-2), transforming growth factor alpha (TGFα), VEGF and combinations thereof. Growth factors are usually added to the culture medium at concentrations ranging between about 1 fg/ml to 1 mg/ml. Concentrations between about 1 to 100 ng/ml are usually sufficient. Simple titration assays can easily be performed to determine the optimal concentration of a particular growth factor.

The biological effects of growth and trophic factors are generally mediated through binding to cell surface receptors. The receptors for a number of these factors have been identified and antibodies and molecular probes for specific receptors are available. LPCs can be analyzed for the presence of growth factor receptors at all stages of differentiation. In many cases, the identification of a particular receptor provides guidance for the strategy to use in further differentiating the cells along specific developmental pathways with the addition of exogenous growth or trophic factors.

Generally, after about 3-10 days *in vitro,* the proliferating LPCs by aspirating the medium, and adding fresh medium to the culture flask. Optionally, the aspirated medium is collected, filtered and used as a condition medium to subsequently passage LPCs. For example the 10%, 20%, 30%, 40% or more condition medium is used.

The LPC cell culture can be easily passaged to reinitiate proliferation. For example after 3-7 days *in vitro,* the culture flasks are shaken well and LPCs are then transferred to a 50 ml centrifuge tube and centrifuged at low speed. The medium is aspirated, the LPCs are resuspended in a small amount of culture medium The cells are then counted and replated at the desired density to reinitiate proliferation. This procedure can be repeated weekly to result in a logarithmic increase in the number of viable cells at each passage. The procedure is continued' until the desired number of LPCs is obtained.

LPCs and LPC progeny can be cryopreserved by any method known in the art until they are needed. *(See, e.g.,* United States patent 5,071, 741, PCT International patent applications WO93/14191, WO95/07611, WO96/27287, WO96/29862, and WO98/14058, Karlsson et al., 65 Biophysical J. 2524-2536 (1993)). The LPCs can be suspended in an isotonic solution, preferably a cell culture medium, containing a particular cryopreservant. Such cryopreservants include dimethyl sulfoxide (DMSO), glycerol and the like. These cryopreservants are used at a concentration of 5-15% *(for example,* 8-10%). Cells are frozen gradually to a temperature of -10°C to -150°C (*for example,* -20°C to -100°C, or -70°C to -80°C).

### Differentiation of Human Liver Progenitor Cells

Depending on the culture conditions, LPCs can be differentiated into hepatocytes, cholangiocytes or sinusoidal cells.

LPCs can be differentiated into hepatocytes, or cholangiocytes by culturing the LPCs on a fixed substrate in a culture medium with HGF and EGF. Alternatively, LPCs can be differentiated into and sinusoidal cells by culturing the LPCs on a fixed substrate in a culture medium with VEGF.

Differentiation of the LPCs can also be induced by any method known in the art which activates the cascade of biological events which lead to growth, which include the liberation of inositol triphosphate and intracellular Ca²⁺, liberation of diacyl glycerol and the activation of protein kinase C and other cellular kinases, and the like. Treatment with phorbol esters, differentiation-inducing growth factors and other chemical signals can induce differentiation. Instead of proliferation-inducing growth factors for the proliferation of LPCs (*see above*), differentiation-inducing growth factors can be added to the culture medium to influence differentiation of the LPCs. Other differentiation inducing growth factors include platelet derived growth factor (PDGF), thyrotropin releasing hormone (TRH); transforming growth factor betas (TGF,s), insulin-like growth factor (IGF-1) and the like.

Differentiated hepatocytes, cholangiocytes or sinusoidal cells are detected using immunocytochemical techniques know in the art. Immunocytochemistry (e.g. dual-label immunofluorescence and immunoperoxidase methods) uses antibodies that detect cell proteins to distinguish the cellular characteristics or phenotypic properties of hepatic cells Cellular markers for hepatocytes and cholangiocytes include albumin and CK10, whereas cellular markers for sinusoidal cells includes Flk. Other suitable markers include glucose 6 phosphatase, glycogen, dipeptidyl peptidase IV, gamma glutaryl transpeptidase

Immunocytochemistry can also be used to identify hepatic cell'ss, by detecting the expression of hepatic genes responsible for liver function such as albumin, alpha 1-antitrpsin, CK-19, alpha fetal protein or human factor VIII.

*In situ* hybridization histochemistry can also be performed, using cDNA or RNA probes specific for the hepatic gene mRNAs. These techniques can be combined with immunocytochemical methods to enhance the identification of specific phenotypes. If necessary, the antibodies and molecular probes discussed above can be applied to Western and Northern blot procedures respectively to aid in cell identification.

### Transplantation of Human Liver Progenitor Cells

Transplantation of new cells into the damaged liver has the potential to repair damaged liver tissue, thereby restoring hepatic function. Optionally fetal stromal cells and or HGF are co-transplanted with the LPCs. However, the absence of suitable cells for transplantation purposes has prevented the full potential of this procedure from being met. "Suitable" cells are cells that meet the following criteria: (1) can be obtained in large numbers; (2) can be proliferated *in vitro* to allow insertion of genetic material, if necessary; (3) capable of surviving indefinitely and facilitate hepatic repair on transplantation in the liver; and (4) are non-immunogenic, preferably obtained from a patient's own tissue or from a compatible donor.

The LPCs obtainable from fetal liver tissue, which are able to divide over extended times when maintained *in vitro* using the culture conditions described herein, meet all of the desirable requirements of cells suitable for liver transplantation purposes and are a particularly suitable cell line as the cells have not been immortalized and are not of tumorigenic origin. The use of LPCs in the treatment of liver disorders can be demonstrated by the use of animal models.

LPCs are administered to any animal with abnormal liver or liver failure symptoms. LPCs can be prepared from donor tissue that is xenogeneic to the host. For xenografts to be successful, some method of reducing or eliminating the immune response to the implanted tissue is usually employed. Thus LPCs recipients can be immunosuppressed, either through the use of immunosuppressive drugs such as cyclosporin, or through local immunosuppression strategies employing locally applied immunosuppressants. Local immunosuppression is disclosed by Gruber, 54 Transplantation 1-11(1992). United States patent 5,026,365 discloses encapsulation methods suitable for local immunosuppression.

As an alternative to employing immunosuppression techniques, methods of gene replacement or knockout using homologous recombination in embryonic stem cells, taught by Smithies et al, 317 Nature 230-234 (1985), and extended to gene replacement or knockout in cell lines (Zheng et al., 88 Proc. Natl. Acad. Sci. 8067-8071 (1991)); can be applied to LPCs for the ablation of major histocompatibility complex (MHC) genes. LPCs lacking MHC expression allows for the grafting of enriched hepatic cell populations across allogeneic, and perhaps even xenogeneic, histocompatibility barriers without the need to immunosuppress the recipient. General reviews and citations for the use of recombinant methods to reduce antigenicity of donor cells are also disclosed by Gruber, 54 Transplantation 1-11(1992). Exemplary approaches to the reduction of immunogenicity of transplants by surface modification are disclosed by PCT International patent application WO 92/04033 and PCT/US99/ 24630. Alternatively the immunogenicity of the graft may be reduced by preparing LPCs from a transgenic animal that has altered or deleted MHC antigens.

LPCs can be encapsulated and used to deliver factors to the host, according to known encapsulation technologies, including microencapsulation (see, *e.g*., United States patents 4,352,883; 4,353,888; and 5,084,350) and macroencapsulation (*see, e.g.,* United States patents 5,284,761, 5,158,881, 4,976,859 and 4,968,733 and PCT International patent applications WO 92/19195 and WO 95/05452). Macroencapsulation is described in United States patents 5,284,761; 5,158,881; 4,976,859; 4,968,733; 5,800,828 and PCT International patent application WO 95/05452. Cell number in the devices can be varied; preferably each device contains between 10³ - 10⁹ cells (for example, 10⁵ to 10⁷ cells). Multiple macroencapsulation devices can be implanted in the host.

LPCs prepared from tissue that is allogeneic to that of the recipient is tested for use by the well-known methods of tissue typing, to closely match the histocompatibility type of the recipient.

LPCs can sometimes be prepared from the recipient's own liver (e.g., in the case of tumor removal biopsies). In such instances the LPCs can be generated from dissociated tissue and proliferated *in vitro* using the methods described above. Upon suitable expansion of cell numbers, the LPCs may be harvested, genetically modified if'necessary, and readied for direct injection into the recipient's liver.

LPCs are administered to the hepatic region can form a hepatic graft, so that the cells form normal connections with neighboring hepatic cells , maintaining contact with transplanted or existing hepaticcells. Thus the transplanted LPCs re-establish the liver tissue which have been damaged due to disease and aging.

Functional integration of the graft into the host's liver tissue can be assessed by examining the effectiveness of grafts on restoring various functions, including blood test for alanine transaminase (ALT), aspartate transaminase (AST), alkaline phosphatase (ALP), albumin, total protein, and total and direct bilirubin.

The ability to expand LPCs *in vitro* for use in transplantation is also useful for *ex vivo* gene therapy. Thus, LPCs provide an additional way to retrieve and expand liver cells for use as vehicles in *ex vivo* gene therapy trials.

### Genetic Modification of Liver Progenitor Cells

Although the LPCs are non-transformed primary cells, they possess features of a continuous cell line. In the undifferentiated state, the LPCs continuously divide and are thus targets for genetic modification. In some embodiments, the genetically modified cells are induced to differentiate into hepatocytes, cholangiocytes or sinusoidal cells by any of the methods described above.

The term "genetic modification" refers to the stable or transient alteration of the genotype of a LPCs by intentional introduction of exogenous DNA. DNA may be synthetic, or naturally deprived, and may contain genes, portions of genes, or other useful DNA sequences. The term "genetic modification" as used herein is not meant to include naturally occurring alterations such as that which occurs through natural viral activity, natural genetic recombination, or the like.

Any useful genetic modification of the cells is within the scope of the present invention. For example, LPCs may be modified to produce or increase production of a biologically active substance such as a growth factor or the like. In one embodiment the biologically active substance is a transcription factor such as a transcription factor that modulates genetic differentiation. In an alternative embodiment the biologically active substance is a non-mitogenic proliferation factor, e.g. v-myc, SV-40 large T or telomerase.

The genetic modification is performed either by infection with viral vectors (retrovirus, modified herpes viral, herpes-viral, adenovirus, adeno-associated virus, and the like) or transfection using methods known in the art (lipofection, calcium phosphate transfection, DEAE-dextran, electroporation, and the like) (*see,* Maniatis et al., in Molecular Cloning: A Laboratory Manual (Cold Spring Harbor Laboratory, N.Y., 1982)). For example, the chimeric gene constructs can contain viral, for example retroviral long terminal repeat (LTR), simian virus 40 (SV40), cytomegalovirus (CMV); or mammalian cell-specific promoters such as tyrosine hydroxylase (TH, a marker for dopamine cells), DBH, phenylethanolamine N-methyltransferase (PNMT), ChAT, GFAP, NSE, the NF proteins (NE-L, NF-M, NF-H, and the like) that direct the expression of the structural genes encoding the desired protein. In addition, the vectors can include a drug selection marker, such as the *E. coli* aminoglycoside phosphotransferase gene, which when co-infected with the test gene confers resistance to geneticin (G418), a protein synthesis inhibitor.

LPCs can be genetically modified using transfection with expression vectors. In one protocol, vector DNA containing the genes are diluted in 0.1X TE (1 mM Tris pH 8.0, 0.1 mM EDTA) to a concentration of 40 µg/ml. 22 µl of the DNA is added to 250 µl of 2X HBS (280 mM NaCl, 10 mM KCl, 1.5 mM Na₂HPO₄, 12 mM dextrose, 50 mM HEPES) in a disposable, sterile 5 ml plastic tube. 31 µl of 2 M CaCl₂ is added slowly and the mixture is incubated for 30 minutes (min) at room temperature. During this 30 min incubation, the cells are centrifuged at 800 g for 5 min at 4°C. The cells are resuspended in 20 volumes of ice-cold PBS and divided into aliquots of 1x10⁷ cells, which are again centrifuged. Each aliquot of cells is resuspended in 1 ml of the DNA-CaCl₂ suspension, and incubated for 20 min at room temperature. The cells are then diluted in growth medium and incubated for 6-24 hr at 37°C in 5%-7% CO₂. The cells are again centrifuged, washed in PBS and returned to 10 ml of growth medium for 48 hr.

LPCs are al'so genetically modified using calcium phosphate transfection techniques. For standard calcium phosphate transfection, the cells are mechanically dissociated into a single cell suspension and plated on tissue culture-treated dishes at 50% confluence (50,000-75,000 cells/cm²) and allowed to attach overnight. In one protocol, the modified calcium phosphate transfection procedure is performed as follows: DNA (15-25 µg) in sterile TE buffer (10 mM Tris, 0.25 mM EDTA, pH 7.5) diluted to 440 µL with TE, and 60 µL of 2 M CaCl² (pH to 5.8 with 1M HEPES buffer) is added to the DNA/TE buffer. A total of 500 µL of 2x HeBS (HEPES-Buffered saline; 275 mM NaCl, 10 mM KCl, 1.4 mM Na₂ HPO₄, 12 mM dextrose, 40 mM HEPES buffer powder, pH 6.92) is added dropwise to this mix. The mixture is allowed to stand at room temperature for 20 min. The cells are washed briefly with 1x HeBS and 1 ml of the calcium phosphate precipitated DNA solution is added to each plate, and the cells are incubated at 37°C for 20 min. Following this incubation, 10 ml of medium is added to the cells, and the plates are placed in an incubator (37°C, 9.5% CO₂) for an additional 3-6 hours. The DNA and the medium are removed by aspiration at the end of the incubation period, and the cells are washed 3 times and then returned to the incubator.

When the genetic modification is for the production of a biologically active substance, the substance can be one that is useful for the treatment of a given liver disorder. LPCs are be genetically modified to express a biologically active agent, such as growth factors, growth factor receptors. For example, it may be desired to genetically modify cells so they secrete a proliferation-inducing growth factor or a differentiation-inducing growth factor. Growth factor products useful in the treatment of liver disorders include HGF, VEGF, FGF-1, FGF-2, EGF, TGFα, TGF,s, PDGF, IGFs, and the interleukins.

The genetically modified LPCs can be implanted for cell therapy or gene therapy into the CNS of a recipient in need of the biologically active molecule produced by the genetically modified cells. Transplantation techniques are detailed below.

Alternatively, the genetically modified LPCs can be subjected to various differentiation protocols *in vitro* prior to implantation. Once the cells have differentiated, they are again assayed for expression of the desired protein. Cells having the desired phenotype can be isolated and implanted into recipients,in need of the protein or biologically active molecule that is expressed by the genetically modified cell.

### Methods for screening effects of drugs on Liver Progenitor Cells

LPCs cultures can be used for the screening of potential therapeutic compositions. For example LPCs are used to identify compounds that effect proliferation, differentiation or survival of liver cells. In addition LPCs are used to identify anti-viral compounds, determine the infectivity of a virus or to identify metabolites of a test compounds. These test compositions can be applied to cells in culture at varying dosages, and the response of the cells monitored for various time periods. Physical characteristics of the cells can be analyzed by observing cell growth and morphology with microscopy. The induction of expression of new or increased levels of proteins such as enzymes, receptors and other cell surface molecules, or of neurotransmitters, amino acids, neuropeptides and biogenic amines can be analyzed with any technique known in the art which can identify the alteration of the level of such molecules. These techniques include immunohistochemistry using antibodies against such molecules, or biochemical analysis. Such biochemical analysis includes protein assays, enzymatic assays, receptor binding assays, enzyme-linked immunosorbant assays (ELISA), electrophoretic analysis, analysis with high performance liquid chromatography (HPLC), Western blots, and radioimmune assays (RIA). Nucleic acid' analysis such as Northern blots can be used to examine the levels of mRNA coding for these molecules, or for enzymes which synthesize these molecules.

LPCs can be used in methods of determining the effect of a biological agent on liver cells. The term "biological agent" refers to any agent, such as a virus, protein, peptide, amino acid, lipid, carbohydrate, nucleic acid, nucleotide, drug, pro-drug or other substance that may have an effect on neural cells whether such effect is harmful, beneficial, or otherwise. Biological agents that are beneficial to hepatic cells are referred to herein as "hepatic agents", a term which encompasses any biologically or pharmaceutically active substance that may prove potentially useful for the proliferation, differentiation or functioning of hepatic cells or treatment of hepatic disease or disorder.

To determine the effect of a potential biological agent on hepatic cells from a a culture of LPCs can be obtained from liver tissue or, alternatively, from a host afflicted with a liver disease or disorder. The choice of culture conditions depends upon the particular agent being tested and the effects one wants to achieve. Once the cells are obtained from the desired donor tissue, they are proliferated *in vitro.*

It is possible to screen for biological agents that increase the proliferative ability of LPCs which would be useful for generating large numbers of cells for transplantation purposes. It is also possible to screen for biological agents that inhibit LPCs proliferation. LPCs are plated in the presence of the biological factors of interest and assayed for the degree of proliferation that occurs. The effects of a biological agent or combination of biological agents on the differentiation and survival of LPCs and their progeny can be determined.

It is possible to screen LPCs which have already been induced to differentiate prior to the screening. It is also possible to determine the effects of the biological agents on the differentiation process by applying them to LPCs prior to differentiation. Generally, the biological agent can be solubilized and added to the culture medium at varying concentrations to determine the effect of the agent at each dose. The culture medium may be replenished with the biological agent every couple of days in amounts so as to keep the concentration of the agent somewhat constant.

Changes in proliferation are observed by an increase or decrease in the number of cells. A "regulatory factor" is a biological factor that has a regulatory effect on the proliferation of LPCs. For example, a biological factor would be considered a "regulatory factor" if it increases or decreases the number of LPCs that proliferate *in vitro* in response to a proliferation-inducing growth factor (such as EGF). Alternatively, the number of LPCs that respond to proliferation-inducing factors may remain the same, but addition of the regulatory factor affects the rate at which the LPCs proliferate. A proliferation-inducing growth factor may act as a regulatory factor when used in combination with another proliferation-inducing growth factor.

Using these screening method, one of skill in the art can screen for potential drug side-effects on hepatic cells by testing for the effects of the biological agents on hepatic cell proliferation and differentiation or the survival and function of differentiated hepatic cells. The proliferated LPCs are typically plated at a density of about 5-10 x 10⁶ cells/ml. If it is desired to test the effect of the biological agent on a particular differentiated cell type or a given make-up of cells, the ratio of hepatocytes and cholangiocyte cells obtained after differentiation can be manipulated by separating the different types of cells.

The effects of the biological agents are identified based upon significant differences relative to control cultures with respect to criteria such as the ratios of expressed phenotypes, cell viability and alterations in gene expression. Physical characteristics of the cells can be analyzed by observing cell morphology and growth with microscopy. The induction of expression of new or increased levels of proteins such as enzymes, receptors and other cell surface molecules, can be analyzed with any technique known in the art which can identify the alteration of the level of such molecules. These techniques include immunohistochemistry using antibodies against such molecules, or biochemical analysis. Such biochemical' analysis includes protein assays, enzymatic assays, receptor binding assays, enzyme-linked immunosorbant assays (ELISA), electrophoretic analysis, analysis with high performance liquid chromatography (HPLC), Western blots, and radioiminune assays (RIA). Nucleic acid analysis such as Northern blots and PCR can be used to examine the levels of mRNA coding for these molecules, or for enzymes which synthesize these molecules.

The factors involved in the proliferation of LPCs and the proliferation, differentiation and survival of LPCs progeny, and their responses to biological agents can be isolated by constructing cDNA libraries from LPCs or LPC progeny at different stages of their development using techniques known in the art. The libraries from cells at one developmental stage are compared with those of cells at different stages of development to determine the sequence of gene expression during development and to reveal the effects of various biological agents or to reveal new biological agents that alter gene expression in liver cells. When the libraries are prepared from dysfunctional tissue, genetic factors may be identified that play a role in the cause of dysfunction by comparing the libraries from the dysfunctional tissue with those from normal tissue. This information can be used in the design of therapies to treat the disorders. Additionally, probes can be identified for use in the diagnosis of various genetic disorders or for use in identifying hepatic cells at a particular stage in development.

The present invention is further illustrated, but not limited, by the following examples.

### EXAMPLE 1: GENERAL METHODS

### Isolation of human fetal liver cells

Permission for the present study was granted from the local ethical committee at Huddinge University hospital. Human FL tissues were obtained from aborted fetuses at 6-9.5. weeks of gestation in accordance with the Swedish guidelines. The study protocol was approved by the local ethics committee. A modified vacuum curettage was performed (33). Gestational age was estimated according to specific anatomical markers (34) in fetuses <12 weeks of gestation and by ultrasound biparietal diameter measurements in older fetuses (35). Gestational age is given as menstrual age. The abortions were performed in pregnancies with no apparent abnormalitie, and no fetuses with anomalies were included. FL was dissected and placed in a sterile tube containing RPMI 1640 medium (Gibco, Invitrogen Corp. UK). The liver was then disintegrated into a single cell suspension by passage through a 70µm metal mesh. The single cell suspension was centrifuged at 200 g for 10 min to pellet the cells. All women donating fetal tissue had been serologically screened for syphilis, toxoplasmosis; rubella, HIV-1, cytomegalovirus, hepatitis B and C, parovirus and herpes simplex types 1 and 2.

### Isolation of cells by magnetic cell sorting and in vitro cultivation

Single cell suspensions were prepared from fetal liver cells in gestation weeks 7-9. Cells were isolated using the human primitive progenitor cell enrichment isolation kit (Stem cell technologies, Vancouver, Canada) followed by the magnetic-activated cell separation magnetic bead system (Stem cell techologies). The method is based on a negative selection of this population using a depletion cocktail including antibodies to 12 lineage-specific cell surface antigens (anti-CD2, -CD3, -CD14, -CD16, -CD19, -CD24, -CD36, -CD38, - CD45RA, -CD56, -CD66b, -glycophorin A). The procedure was carried out as described by the manufacturers. On every occasion, the recovered progenitor cell's were immediately analysed by the flow cytometer to make sure that no contaminating lin+ cells were present and to confirm the progenitor phenotype (CD117+/CD34+/lin-) of the cells. The recovered progenitor cells at the end of the procedure were tested for viability, afterwhich the cells were seeded in plastic petri dishes coated with collagen type I (Biocoat, Becton and Dickinson, New Jersey, USA) and cultivated in Dulbecco's Modified Eagle Medium (DMEM) (GIBCO, Invitrogen, Stockholm, Sweden) containing, 10% inactivated fetal calf serum, penicillin and streptomycin, 5%L-glutamine, 5%minimum essential amino acids, 50ng/ml HGF (R&D Systems, Abingdon, England), 20ng/ml EGF (R&D Systems) and 10 ng/ml basic fibroblast growth factor (R&D Systems). Every third day the medium was collected, centrifuged, sterile filtered and used as conditioned medium (CM). All subsequent subculturing was performed using 20%CM. In some experiments, the progenitor cells were grown in DMEM medium containing 20ng/ml HGF, 10ng/ml EGF and 50ng/ml of VEGF (R&D Systems) and allowed to divide in culture. For detection of proliferation, cells in culture were incubated with the thymidine analogue BrdU (30mM) for 30 min. Cells were washed and stained for albumin, cytokeratin 19 and BrdU using, a FITC-conjugated goat antibody against human albumin (Natutec, Frankfurt, Germany), a non-conjugated anti-human cytokeratin 19 (Neomarker, USA), and a non-conjugated anti-BrdU antibody (Sigma, Stockholm, Sweden). Other antibodies used for phenotyping were anti-CD45, -CD14, -CD90, -CD117, -CD34 (Pharmingen, USA), -Flk-1 (ReliaTech, Germany), and secondary subclass specific antibodies goat-anti-mouse IgG1 (FITC/Texas red) and goat-anti-mouse IgG2a (FITC/Texas red). Flow cytometry and immunocytochemistry was used to phenotype the progenitor cells. The procedures were carried out as described (36).

Freshly isolated progenitor cells (P0) and *in vitro* expanded cells in passages 6 (P6) and 12 (P12) were used for transplantation studies.

### Mice

The animal care and use committee at Huddinge hospital approved of the animal protocols. Liver injury was induced in C57 black/nude mice (n=16) by administration of GalN (Sigma Chemicals Co., Stockholm, Sweden) intraperitonially at 0.7 g/kg body weight, 24 hrs before partial hepatectomy. GalN was dissolved in phosphate-buffered saline, pH 7.4 (PBS) at 100 mg/ml. Partial hepatectomy (PH) was carried out as described earlier (37). Administration of GalN was continued for ten days after PH.

Hepatic progenitor cells were transplanted into the spleen of these animals. Animals were anaesthetized under ether and typically 1x10₅ freshly isolated cells (P0) and 1x10₆ cells in passages 6 and 12 suspended in 200ul of DMEM medium were injected into the spleen over approximately 10-15s. Four mice were sham-transplanted with just DMEM medium. After securing hemostasis, the abdominal incision was closed and the animals were monitored closely until recovery.

### Preparation of livers and analysis of fluorescence in cryosections

Mice were killed 4 weeks after transplantation and the livers, spleens and lungs were excised. Two or three biopsies from each liver of approximately 2 mm₂ were shock frozen in liquid nitrogen and used for RNA isolation to perform RT-PCR analysis. The rest of the liver tissue was shock frozen for fluorescence and immunohistochemical analysis. Cryosections 5 µm in thickness were air dried and fixed with cold 30% acetone in methanol for 10 min and further analysed by immunohistochemistry.

### Immunohistochemistry

We initially tested two methods for the detection of human cells in the mouse parenchyma; a) An *in situ* hybridization technique using a digoxygenin labeled total human DNA probe (Cytocell, Oxfordshire, UK) (38) and b) a mouse anti-human nuclei monoclonal antibody (Chemicon, CA, USA) followed by staining with biotinylated horse-anti-mouse secondary antibody. The immunoperoxidase procedure was carried out using Vectastain Elite ABC kit (ImmunKemi, Stockholm, Sweden) as descibed by the manufacturers. The diaminobenzidine tetrahydrochloride (DAB)-Nickel substrate kit was used as color developer. For doublestainings, combinations of DAB (gives brown colour staining) and/or DAB-Ni (black) and/or the Vector NovaRed kit were used. Other primary antibodies used were, a FITC-conjugated goat antibody against human albumin (not cross-reactive with mouse) (Natutec, Frankfurt, Germany), a non-conjugated anti-human cytokeratin 19 (Neomarker, USA), and a nonconjugated mouse-anti-human CD26 (detects dipeptidyl peptidase IV) (Pharmingen, USA). GGT , G-6-P and glycogen were demonstrated *in situ* as described earlier (39, 40). Sections were counterstained with hematoxylin and mounted in mounting media (ImmunKemi, Stockholm, Sweden).

### Morphometric analysis

60 serial sections of each mouse liverwere screened for DAB-Ni-positive human cells. The number of transplanted cells were determined in clusters of three sizes i.e. cells arranged singly or in clusters of ≥ 2-20 or > 20 cells each. We analyzed a minimum of 100 high-power fields in tissues from all transplanted animals.

### Reverse transcriptase-polymerase chain reaction (RT-PCR)

Total RNA was extracted from four human-mouse chimeric murine liver tissues and' one normal mouse liver tissue, using the Micro-FastTrack RNA isolation kit (Invitrogen, Groningen, The Netherlands). We used human specific primers to detect human albumin, CK19, α-fetoprotein and α1-antitrypsin expression in the mouse liver. Primers were selected for CK-19, α-fetoprotein, albumin, antitrypsin and glucose-6-phosphate dehydrogenase (G6PD) by using the Primer Express software version 2.0 (Applied Biosystems). Each set of primers was designed to target cDNA alone, not contaminating DNA. Primer sets were commercially synthesized by CyberGene (Huddinge, Sweden).

Primer sequences were
"CK-19- sense":5'-CCTGCGGGACAAGATTCTTG-3' (SEQ ID NO:1),
antisense- 5'-ACGGGCGTTGTCGATCTG-3' (SEQ ID NO:2), expected product size (bp):70
"a-fetoprotein- sense": 5'-GCAAAGCTGAAAATGCAGTTGA-3'(SEQ ID NO:3),
antisense- 5'-GGAAAGTTCGGGTCCCAAAA-3' (SEQ ID NO:4), expected product size (bp):129
"albumin-sense": 5'-GCTTTGCCGAGGAGGGTAA-3'(SEQ ID NO:5),
antisense- 5'-GGTAGGCTGAGATGCTTTTAAATGT-3', expected product size (bp):88
"α1-antitrypsin-sense": 5'-CAGAGGAGGCACCCCTGAA-3'(SEQ ID NO:6),
antisense-5'-AGTCCCTTTCTCGTCGATGGT-3'(SEQ ID NO:7), expected product size (bp):71
"G6PD-sense": 5'-TGC CCC CGA CCG TCT AC-3'(SEQ ID NO:8),
Antisense-5'-ATG CGG TTC CAG CCT ATC TG-3'(SEQ ID NO:9), expected product size (bp):76.

PCR reactions were done in duplicates in 96-well optical plates in a total volume of 25 µL. Each reaction contained 2.5 µL of cDNA, 12.5 µL SYBR Green Master Mix (Applied Biosystems), and 500nM of each primer. Positive and' negative controls were included in all runs. Thermal cycling conditions were 2 min at 50°C initially and 10 min at 95°C, as recommended by the manufacturer. Cycle conditions were 40 cycles at 95°C for 15s and at 60°C for 1 min. The housekeeping gene, G6PD was included as endogenous normalization control, which was used to confirm successful RNA isolation and reverse transcription, and the total amount of RNA in every sample. To visualize the results from the RT-PCR, we ran the PCR products on a ready-to-use 12.5% nondenaturing polyacrylamide gel electrophoresis system and the bands were stained by automated silverstaining (Pharmacia Biotech, Uppsala, Sweden).

### Statistical methods

The data are presented as mean±SD. The significance of differences was analyzed with the Student's t and analysis of variance (ANOVA). A *p* value of less than 0.05 was considered to be significant.

### EXAMPLE 2 CD117+/CD34+/LIN- LIVER PROGENITOR CELLS CAN DIFFERENTIATE INTO HEPATOCYTES AND CHOLANGIOCYTES IN VITRO

Using a kit designed to isolate primitive hematopoietic progenitors, a population of cells from human fetal livers (gw 6-9) were otained that that did not express any committed hematopoietic markers. Further phenotyping of this population showed expression of the stem cell markers CD117 and CD34 but no expression of liver markers such as albumin (hepatocyte marker) and CK19 (cholangiocyte marker). Nor was there any expression of Thy-1 (CD90) or CD45 (Fig. 1a). This population represent approximately 0.5%-0.7% of whole fetal livers in gestation weeks 6-9. The expressions of CD45 and CD90 were not observed during subculture of the cells. Upon cultivation, it was found these cells to be a mixture of both adherent (∼85%) (Fig. 1b) and non-adherent populations (∼15%). The non-adherent population could not be expanded further under culture conditions given below. CD117+/CD34+/Lin- cells and their progeny tend to grow in colonies (Fig. 1b). The first marker to be expressed by the adherent progenitor cells after 2 days in culture was c-Met (hepatocyte growth factor receptor) (Fig. 1b). *In vitro* cultivation of these cells for two weeks in culture medium containing hepatocyte growth factor (HGF) and epidermal growth factor (EGF), showed the presence of four types of cells: i) ∼85% double positive cells expressing albumin and CK19, ii) ∼4% double negative cells, iii) ∼6% single positive cells expressing only albumin and iv) ∼5% single positive cells expressing only CK19 (Fig. 1c). This phenotype was maintained for several passages during cultivation (Fig. 1c). However, from P11 onwards there was a slight decrease in the numbers of double positive cells (∼60%) and double negative (∼3%), while single positive cells for albumin or CK19 increased. These data demonstrate that non-hematopoietic primitive progenitor cells from early developing livers can differentiate *in vitro* into hepatocytes and cholangiocytes.

### EXAMPLE 3: CD117+/CD34+/LIN- LIVER PROGENITOR CELLS DIFFERENTIATE INTO LIVER SINUSOIDAL ENDOTHELIAL CELLS IN VITRO IN THE PRESENCE OF VASCULAR ENDOTHELIAL GROWTH FACTOR

Interestingly, when adherent CD117+/CD34+/Lin- cells were allowed to differentiate in culture medium containing 50ng/ml vascular endothelial growth factor (VEGF), we observed a large proportion of cells with endothelial-like morphology. Further characterization of this cell population using liver cell markers including Flk-1 known to be expressed on fetal liver endothelial progenitors, revealed four populations of cells a) endothelial cells expressing the receptor Flk-1 (∼50%), b) hepatocytes (∼13%), c) cholangiocytes (∼17%)(Fig. 1d), and d) a cell population that did not express any of these markers (∼20%) (data not shown). the sinusoidal phenotype of the Flk-1+ cells was confirmed by using a vast panel of antibodies (Table 1). Human umbilical vein endothelial cells were used to demonstrate the phenotypic differences between vascular and sinusoidal endothelial cells (Table 1). Electron microscopic analysis revealed the presence of fenestrae and absence of a basement membrane characteristic of sinusoidal endothelial cells (data not shown). These data show that nonhematopoietic primitive progenitor cells from early developing livers can differentiate *in vitro* into hepatocytes, cholangiocytes and endothelial cells.

**Table 1. Cell yield and viability of magnetically isolated and cultured primitive progenitor cells from fetal livers in the first trimester.**

| Embryonic age of fetal liver | Total no. of cells | No. of CD117+/ CD34+/Lin- | Viability | Average doubling time | No. of cell subpassages obtained | Approximate no. of cells obtained |
|---|---|---|---|---|---|---|
| 6 weeks | 6x10⁶ | 3.1x10⁴ | 93% | 12-15 days | 4 | ∼5x105 |
| | | | | | | |
| 6 weeks | 5.6x10⁶ | 2x10⁴ | 95% | 7-10 days | 4 | ∼4.5x105 |
| | | | | | | |
| 6.5 weeks | 10x10⁶ | 4,5x10⁴ | 94% | 4-5 days | 12* | ∼400x106 |
| 6.5 weeks | 6.8x10⁶ | 2,5x10⁴ | 97% | 4-5 days | 14* | ∼360x106 |
| 8 weeks | 8x10⁶ | 3.2x10⁴ | 92% | 10-12 days | 2 | ∼7x104 |
| | | | | | | |
| 8.5 weeks | 15x10⁶ | 6.5x10⁴ | 90% | 4-5 days | 12* | ∼550x106 |
| 8 weeks | 7x10⁶ | 3.2x10⁴ | 95% | 12-14 days | 2 | ∼8x105 |
| | | | | | | |
| 8.5 weeks | 18x10⁶ | 6.5x10⁴ | 92% | 2-3 days | 10* | ∼70x106 |
| 9.5 weeks | 13x10⁶ | 5.5x10⁴ | 97% | 8-10 days | 9* | ∼50x106 |
| | | | | | | |
| 9 weeks | 11x10⁶ | 7.5x10⁴ | 94% | 3-4 days | 12* | ∼307x106 |
| 9 weeks | 5.6x10⁶ | 4.5x10⁴ | 93% | 8-10 days | 2 | ∼10x104 |
| | | | | | | |
| | | | | | | |
| 9.5 weeks | 10.5x10⁶ | 7.5x10⁴ | 92% | 4-5 days | 12* | ∼320x106 |
| 9.5 weeks | 17.6x10⁶ | 8.8x10⁴ | 95% | 5-7 days | 12 | ∼360x106 |
| 9 weeks | 14x10⁶ | 13.3x10⁴ | 96% | 3-4 days | 11 | ∼272x106 |
| 9.5 weeks | 15.5x10⁶ | 9.5x10⁴ | 97% | 12-14 days | 4 | ∼389x106 |
| | | | | | | |
| 9.5 weeks | 13.6x10⁶ | 12x10⁴ | 91% | 4-5 days | 11 | ∼245x106 |

| | | | | | | |
|---|---|---|---|---|---|---|
| *Cells still in culture at present. | | | | | | |

### EXAMPLE 4: LIVER PROGENITOR CELLS CAN BE PASSAGED EXTENSIVELY IN VITRO

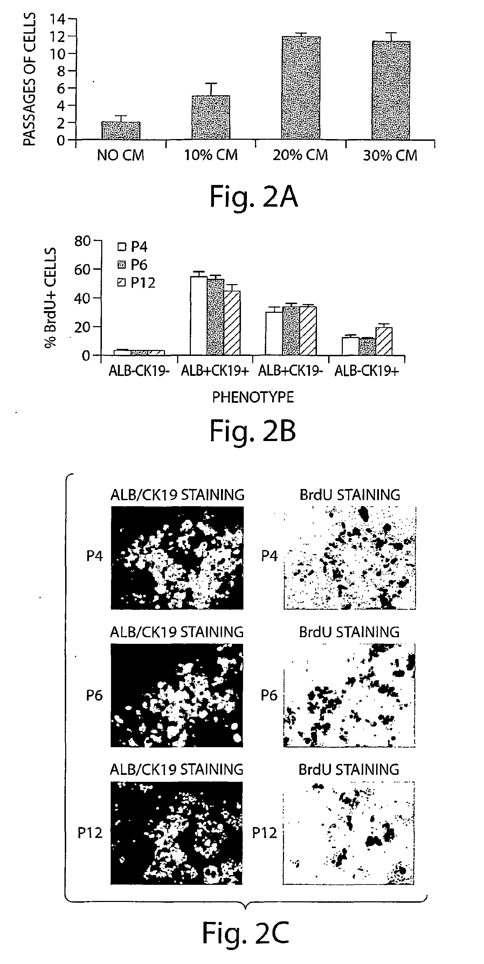

The proliferation of the liver progenitors was dependent on the regular use of 20% conditioned medium (CM) during cultivation. Using 20% CM, these cells can be cultured to at least 12 passages. Removal of the CM significantly decreased the number of cell passages obtained (*p*<0.001, Fig. 2a), reflecting a decrease in the proliferation of the cells. It was found that a high proliferative capacity was observed in the double positive cells (ALB+CK19+) and the single albumin positive cells (ALB+CK19-) as detected by the incorporation of BrdU (Fig. 2b&c). The cells can be maintained for long periods with stable phenotype. I t is important to mention that, not all FLs generated cells which proliferated rapidly and which could be maintained in culture for several passages (Table 2). Successful proliferation for long periods was dependent on the quality of FL tissue obtained. However, it was found that CD117+/CD34+/Lin- cells isolated from all FLs tested differentiated into hepatic cells. Data from the *in vitro* studies demonstrated that CD117+/CD34+/Lin- cells and their progeny have extensive replication capacity and can be maintained for long periods with a stable phenotype.

**Table 2. Phenotypic characteristics of Flk-1+cells obtained from liver progenitors**

| **Antibodies to** | **HUVEC** | **Flk-1+** |
|---|---|---|
| **CD 141** | + | + |
| ***CD 142** | + | + |
| **CD 144** | + | - |
| **Acetylated LDL** | + | + |
| **Ulex Europaeus** | + | - |
| ***CD 106** | + | + |
| **CD 62E** | + | - |
| **CD31** | + | - |
| **VWF** | + | - |
| **CD 105** | + | + |
| **Fibroblast** | - | - |
| **Alpha-actin** | - | - |

| | | |
|---|---|---|
| ***Expressed only on activated endothelial cells. HUVEC: Human umbilical vein endothelial cells.** | | |

### EXAMPLE 5: EXPANDED HUMAN FETAL PROGENITOR CELLS SUCCESSFULLY ENGRAFT AND DIFFERENTIATE INTO MATURE HEPATOCYTES, CHOLANGIOCYTES AND SINUSOIDS IN THE LIVERS OF GALN-TREATED MICE

To test whether freshly isolated CD117+/CD34+/Lin- cells (P0) and cells expanded in culture (P6 and P12) have the potential to differentiate and be functional *in vivo*, these cells were transplanted into mice that were first partially hepatectomized and then treated with Dgalactosamine (GalN) to induce acute liver injury. This protocol induces acute liver injury and facilitates hepatic regeneration (21). Two mice in the control and two in the test group died within 24 hrs after treatment. Results from mice surviving at four weeks after cell transplantation are presented. After intrasplenic transplantation, primary P0 cells, as well as P6 and P12 subpassaged cells, survived in the GalN-treated mouse liver. Experiments to compare the results obtained using a human centromere probe and an antihuman nuclei antibody to localize transplanted cells gave similar results. The species specificity of the human DNA probe (Fig. 3a&b) and the anti-human nuclei antibody is demonstrated by the positive result with human liver (Fig. 3c) and the negative immunohistochemistry with sham-transplanted livers from nude mice (Fig. 3d). Freshly isolated CD117+/CD34+/lin- cells when transplanted, differentiated into hepatocytes (Fig. 3e), sinusoidal cells (Fig. 3f) and formed bile ducts (Fig. 3g) at four weeks after transplantation. Hepatic progenitor cells in P6 and P12 when transplanted engrafted and reconstituted the acutely damaged liver (Fig. 3h-j). Transplanted cells were found in the livers of the mice (Fig. 3k), but not in other tissues such as spleen (Fig. 3I) and lung (Fig. 3m) and in sham-transplanted animals (Fig. 3n). The human transplanted cells expressed hepatocyte markers such as glucose-6-phosphatase (G-6-P) (Fig. 4a) and glycogen (Fig. 4b), and biliary markers such as dipeptidyl peptidase IV (DPPIV) (Fig. 4c) and gamma glutamyl transpeptidase (GGT) (Fig. 4d). In three mice, segments of regenerating tissue that was 90% repopulated by human liver cells was found (Fig. 4e). Clear areas of bile ducts completely repopulated by human progenitors wer also observed. These cells stained positively for CK19 and the anti-human nuclei antibody (Fig. 4f). Monoclonal antibody against human albumin which does not cross react with murine albumin was used to examine the expression of human albumin in the transplanted cells. The negative immunohistochemistry with sham transplanted livers from nude mice (Fig. 4g) showed the species specificity of the antibody. Several albumin positive structures (Fig. 4h-j) in all of the 10 transplanted mice were found, however w foci expressing human albumin in sham-transplanted mice (*n*=2) was not observed using identical conditions. Human albumin was detected only in the livers of the mice, but not in the spleen or lungs. As a note of interest no tumor formations were observed in any of the mice analyzed.

To determine whether liver engraftment was comparable between human hepatic progenitors in P0, P6 and P12, 60 serial sections of each mouse liver were screened for DAB-Ni-positive human cells. The number of transplanted cells in clusters of three sizes i.e. cells arranged singly or in clusters of ≥5-20 or >20 cells was determined. The analysis showed that there was no significant difference in the number of transplanted cells arranged singly or in clusters of ≥2-20 cells between the animals receiving P0, P6 and P12 cells. No clusters of >20 cells were observed in mice transplanted with P0 cells, but were observed in mice that received cells in P6 and P12 (Table 3) (*p*<0.001, ANOVA). In mice transplanted with P0 cells we detected a ten-fold increase in the number of human transplanted cells while in mice injected with P6 and P12 cells a six-fold increase was detected (Table 3). These data demonstrate that freshly isolated CD117+/CD34+/Lin- cells successfully proliferate and differentiate *in vivo* into mature hepatocytes cholangiocytes and sinusoidal endothelial cell's. In addition, *in vitro* expanded CD117+/CD34+/Lin- cells and their progeny proliferate *in vivo* and successfully reconstitute the damaged mouse liver.

**Table 3. Human liver progenitor cell engraftment in D-GalN-treated mice**

| **Detection of transplanted cells four weeks after transplantation** | | | | | |
|---|---|---|---|---|---|
| **Cell passage no.** | **No. of cells transplanted** | **Human cells detected (0.6 cm³ total liver volume after partial hepatectomy)** | **% of single cells** | **% of clusters of ≥2-20 cells** | **% of clusters of >20 cells** |
| **P0** | **1x10⁵** | **1.0±0.2x10⁶** | **58** | **42** | **0** |
| **P0** | | | **60** | **40** | **0** |
| **P6** | | | **57** | **41** | **2** |
| **P6** | **1x10⁶** | **6.7±2.3 x 10⁶** | **50** | **45** | **5** |
| **P6** | | | **51** | **47** | **2** |
| **P6** | | | **52** | **45** | **3** |
| **P12** | | | **63** | **35** | **2** |
| **P12** | **1.10⁶** | **6.1±2.2 X10⁶** | **57** | **40** | **3** |
| **P12** | | | **62** | **33** | **5** |
| **P12** | | | **58** | **40** | **2** |

### EXAMPLE 6:TRANSCRIPTION OF HUMAN LIVER-SPECIFIC GENES IN MICE TRANSPLANTED WITH HUMAN HEPATIC PROGENITOR CELLS

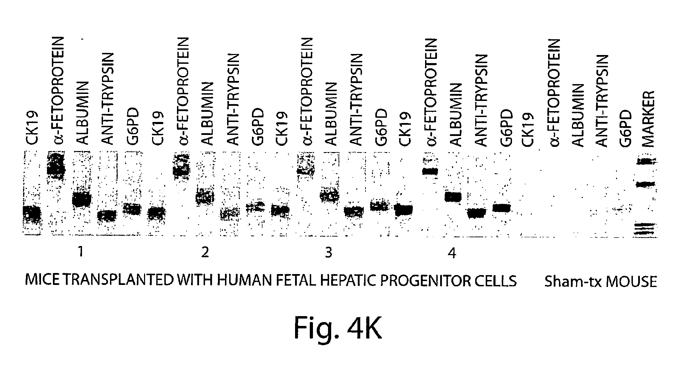

The engraftment of the transplanted cells was conformed by determining the expression of human genes in the transplanted mice. Livers of the mice sacrificed were analyzed one month after transplantation of human fetal hepatic progenitor cells by RT-PCR using primers specific for human liver-specific genes, including albumin, α1-antitrypsin, CK19, and AFP. RNA from the liver of a sham-transplanted nude mouse resulted in amplification for G6PD which was used as a control for the integrity of the RNA. The CK19, albumin, and AFP primers were species specific for human as they did not amplify the respective mouse genes, however α1-antitrypsin was not found to be species specific (Fig. 4k). These results show that both freshly isolated (P0) and cells in various passages P6, and P12 engraft the damaged mouse liver.

### EXAMPLE 7: PROTECTION OF HUMAN FACTOR VIII IN MICE TRANSPLANTED WITH LIVER PROGENITOR CELLS

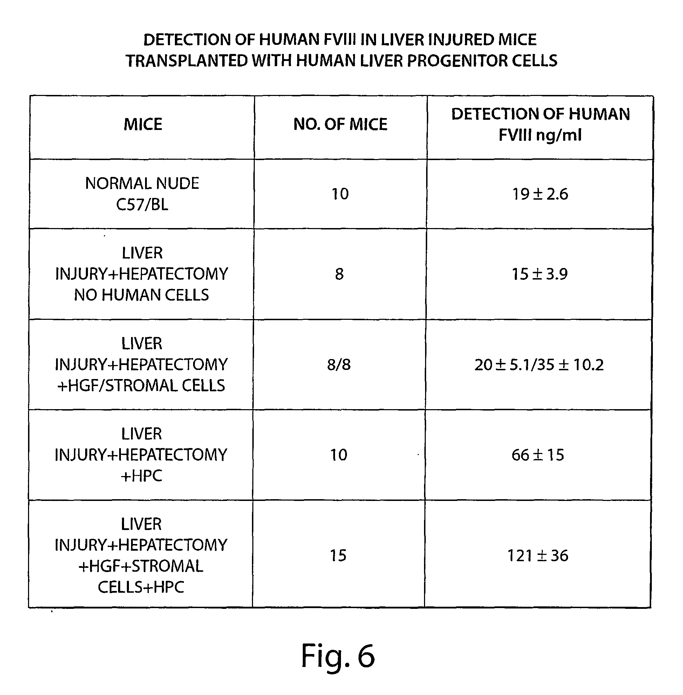

Levels of human FVIII are negligible in chemically liver-injured and hepatectomized mice, as well as mice treated in the same manner and in addition received the growth factor HGF and/or stromal cells. However, chemically liver-injured and hepatectomized mice that received only human liver progenitor cells or a combination of HGF+stromal+progenitor cells demonstrated high levels of human FVIII in their plasma. (Figure 6)

## Claims

1. An isolated cell population consisting ofcells derived from liver tissue of a human which are
a. CD117⁺, CD34⁺, CD45⁻ and Lin⁻;
b. capable of proliferating in a culture; and
c. capable of differentiating *in vivo* into a hepatocyte, a cholangiocyte and a sinusoidal cell.

2. The isolated cell population of claim 1, wherein the population is capable of doubling at least 6 times.

3. The isolated cell population of claim 1, wherein the population is capable of doubling at least 12 times.

4. The isolated cell population of claim 1, wherein said population is an adhesion culture.

5. A method of producing a human liver sinusoidal cell *in vitro* comprising
a. providing a cell suspension comprising a CD117⁺, CD34⁺, and Lin⁻ cell;
b. culturing the cell suspension; and
c. differentiating the cell progeny in a culture medium containing vascular endothelial growth factor.

6. A method of producing a human liver hepatocyte, or cholangiocyte *in vitro* comprising
a. providing a cell suspension comprising a CD117⁺, CD34⁺, and Lin⁻ cell;
b. culturing the cell suspension; and
c. differentiating the cell progeny in a culture medium containing EGF and HGF.

7. A method of producing a population of human liver progenitor cells which differentiate *in vivo* into hepatocytes, cholangiocytes, and sinusoidal cells, comprising selecting from a population of human liver derived cells for cells that are CD117⁺, CD34⁺, CD45⁻and Lin⁻.

8. Use of multipotent liver progenitor cell comprising
providing an *in* vitro cell culture comprising multipotential human CD117⁺, CD34⁺, CD45⁻ and Lin⁻ liver progenitor cells wherein said cells maintain multipotential capacity to differentiate into hepatocytes, cholangiocytes or sinusoidal cells, for the manufacture of a composition for transplanting into a host.

9. The use of claim 8, wherein transplantation further comprises co-transplanting fetal liver stromal cells or fetal liver mesenchymal cells in said host, disclaiming the use of human embryos.

10. The use of claim 8, wherein transplantation further comprises administering to said host hepatocyte growth factor.

## Patentansprüche

1. Isolierte Zellenpopulation bestehend aus Zellen, die aus Lebergewebe eines Menschen stammen, wobei die Zellen
a. CD117⁺, CD34⁺, CD45⁻ und Lin⁻ sind;
b. in der Lage sind, in einer Kultur zu proliferieren; und
c. in der Lage sind, sich in vivo in einen Hepatozyten, einen Cholangiozyten und eine sinusoidale Zelle zu differenzieren.

2. Isolierte Zellenpopulation nach Anspruch 1, wobei die Population in der Lage ist, sich mindestens sechs Mal zu verdoppeln.

3. Isolierte Zellenpopulation nach Anspruch 1, wobei die Population in der Lage ist, sich mindestens zwölf Mal zu verdoppeln.

4. Isolierte Zellenpopulation nach Anspruch 1, wobei die Population eine Adhäsionskultur ist.

5. Verfahren zur Herstellung einer menschlichen sinusoidalen Leberzelle in vitro, aufweisend
a. Bereitstellen einer Zellensuspension, die eine CD117⁺⁻, CD34⁺-, und Lin⁻-Zelle aufweist;
b. Kultivieren der Zellensuspension; und
c. Differenzieren der Zellen-Nachkommenschaft in einem Kulturmedium, das Gefäßendothel-Wachstumsfaktor enthält.

6. Verfahren zur Herstellung eines menschlichen Leberhepatozyten oder Lebercholangiozyten in vitro, aufweisend
a. Bereitstellen einer Zellensuspension, die eine CD117⁺-, CD34⁺-, und Lin⁻-Zelle aufweist;
b. Kultivieren der Zellensuspension; und
c. Differenzieren der Zellen-Nachkommenschaft in einem Kulturmedium, das EGF und HGF enthält.

7. Verfahren zur Herstellung einer Population von menschlichen Leber-Vorläuferzellen, die sich in vivo in Hepatozyten, Cholangiozyten und sinusoidale Zellen differenzieren, aufweisend ein Auswählen aus einer Population von Zellen, die aus einer menschlichen Leber stammen, hinsichtlich Zellen, die CD117⁺, CD34⁺, CD45⁻ und Lin- sind.

8. Verwendung multipotenter Leber-Vorläuferzellen, aufweisend Bereitstellen einer in vitro-Zellkultur, die multipotente menschliche CD117⁺-, CD34⁺-, CD45⁻- und Lin⁻-Leber-Vorläuferzellen aufweist, wobei die Zellen die Multipotenzfähigkeit, sich in Hepatozyten, Cholangiozyten oder sinusoidale Zellen zu differenzieren, beibehalten, zur Herstellung einer Zusammensetzung zur Transplantation in einen Wirt.

9. Verwendung nach Anspruch 8, wobei die Transplantation außerdem ein Co-Transplantieren von fetalen Leber-Stromazellen oder fetalen Leber-Mesenchymzellen in den Wirt aufweist, wobei die Verwendung menschlicher Embryonen nicht beansprucht wird.

10. Verwendung nach Anspruch 8, wobei die Transplantation außerdem ein Verabreichen von Hepatozyten-Wachstumsfaktor an den Wirt aufweist.

## Revendications

1. Population cellulaire isolée constituée de cellules issues de tissu hépatique d'un humain et qui sont
a. CD117⁺, CD34⁺, CD45⁻ et Lin⁻ ;
b. capables de proliférer dans une culture ; et
c. capables de se différencier in vivo en hépatocytes, cholangiocytes et cellules sinusoïdales.

2. Population cellulaire isolée selon la revendication 1, laquelle population est capable de doubler au moins 6 fois.

3. Population cellulaire isolée selon la revendication 1, laquelle population est capable de doubler au moins 12 fois.

4. Population cellulaire isolée selon la revendication 1, laquelle population est une culture par adhésion.

5. Procédé pour produire une cellule sinusoïdale hépatique humaine in vitro, consistant à :
a. se procurer une suspension cellulaire comprenant des cellules CD117⁺, CD34⁺ et Lin⁻ ;
b. cultiver la suspension cellulaire ; et
c. différencier la descendance cellulaire dans un milieu de culture contenant du facteur de croissance endothélial vasculaire.

6. Procédé pour produire un hépatocyte ou cholangiocyte hépatique humain in vitro, consistant à :
a. se procurer une suspension cellulaire comprenant des cellules CD117⁺, CD34⁺ et Lin⁻ ;
b. cultiver la suspension cellulaire ; et
c. différencier la descendance cellulaire dans un milieu de culture contenant de l'EGF et du HGF.

7. Procédé pour produire une population de cellules souches hépatiques humaines qui se différencient in vivo en hépatocytes, cholangiocytes et cellules sinusoïdales, comprenant la sélection, à partir d'une population de cellules issues de foie humain, des cellules qui sont CD117⁺, CD34⁺, CD45⁻ et Lin⁻.

8. Utilisation de cellules souches hépatiques multipotentes, comprenant l'opération consistant à se procurer une culture cellulaire in vitro comprenant des cellules souches hépatiques humaines multipotentes CD117⁺, CD34⁺, CD45⁻ et Lin⁻, lesdites cellules conservant une capacité multipotente à se différencier en hépatocytes, cholangiocytes ou cellules sinusoïdales, pour la fabrication d'une composition destinée à une transplantation dans un hôte.

9. Utilisation selon la revendication 8, dans laquelle la transplantation comprend en outre la co-transplantation de cellules stromales hépatiques foetales ou de cellules mésenchymateuses hépatiques foetales dans ledit hôte, en excluant l'utilisation d'embryons humains.

10. Utilisation selon la revendication 8, dans laquelle la transplantation comprend en outre l'administration audit hôte de facteur de croissance des hépatocytes.
